# EUROPÄISCHE PATENTSCHRIFT

(11) **EP 4 592 669 B1**
(45) Veröffentlichungstag und Bekanntmachung des Hinweises auf die Patenterteilung: **31.12.2025**
(21) Anmeldenummer: 24153448.6
(22) Anmeldetag: 23.01.2024
(51) Int. Cl.: G01N 21/31, G01N 15/06, G01N 21/47, G01N 21/53, G01N 21/94, G01N 15/00, G01N 21/15, G01N 15/075, G01N 21/359, G01N 21/51

(54) **OPTO-MECHANISCHES ANALYSEGERÄT ZUR BESTIMMUNG VON FEINSTAUB IN EINEM MESSGAS**
OPTO-MECHANICAL ANALYSER FOR DETERMINING FINE DUST IN A MEASUREMENT GAS
APPAREIL D'ANALYSE OPTOÉLECTRONIQUE DESTINÉ À LA DÉTERMINATION DE POUSSIÈRES FINES DANS UN GAZ DE MESURE

(43) Veröffentlichungstag der Anmeldung: 30.07.2025
(73) Patentinhaber: Endress+Hauser SICK GmbH+Co. KG, 01458 Ottendorf-Okrilla (DE)
(72) Erfinder: SCHLADITZ, Alexander, 01127 Dresden (DE); REGEHR, Jürgen, 01309 Dresden (DE); URBAN, Titus, 01127 Dresden (DE)
(74) Vertreter: Manitz Finsterwald Patent- und Rechtsanwaltspartnerschaft mbB

(56) Entgegenhaltungen:
- EP-A2- 2 500 710
- CN-A- 110 095 477
- CN-U- 205 317 630
- JP-A- 2022 138 630
- US-A- 3 868 184
- US-A- 5 751 423
- US-A1- 2022 120 679

## Beschreibung

Die Erfindung betrifft ein Opto-mechanisches Analysegerät zur Bestimmung von Feinstaub in einem Messgas, wobei kontinuierlich die Genauigkeit dieser überprüft werden kann.

Unter Feinstaub werden kleinste Partikel verstanden, deren Größe (aerodynamische Durchmesser) weniger als 10 µm beträgt.

Feinstaub wird charakterisiert mit PM₁₀, PM_{2,5} und PM₁. Die als Feinstaub PM₁₀ bezeichnete Staubfraktion enthält quasi ausschließlich Teilchen mit einem Durchmesser <10 µm. Die als Feinstaub PM_{2,5} bezeichnete Staubfraktion enthält quasi ausschließlich Teilchen mit einem Durchmesser <2,5 µm. PM_{2,5} ist somit eine Teilmenge von PM₁₀. PM₁ ist analog zu PM_{2.5} bzw. PM₁₀ zu verstehen.

Feinstaub ist besonders kritisch für die öffentliche Gesundheit, da insbesondere die kleinsten Partikel (<1µm) tief in die Lungen eindringen und sogar in den Blutkreislauf gelangen können. Dies kann zu einer Vielzahl von Gesundheitsproblemen führen, darunter Atemwegserkrankungen, Herz-Kreislauf-Erkrankungen und Lungenkrebs. Die Überwachung und Verringerung von Feinstaubemissionen ist daher ein wichtiger Bestandteil der Umweltpolitik und des Gesundheitsschutzes. Die Anforderungen an die Bestimmung von Feinstaub nehmen ständig zu, denn die Filteranlagen von emittierenden Anlagen, wie Kohlekraftwerke, werden immer besser, sodass die Konzentrationen sinken, wobei gleichzeitig die erlaubten Emissionsgrenzwerte schärfer werden.

Der Feinstaub, der in einem Messgas enthalten ist, kann mit entsprechenden Analysegeräten erfasst werden. Problematisch ist, dass das Messgas teilweise hohe Temperaturen aufweist und entsprechende Analysegeräte daher regelmäßig gewartet und überprüft werden müssen.

Die EP 2 500 710 A2 zeigt ein Streulichtmessgerät, insbesonders zur kontinuierlichen Messung der Partikelkonzentration im Abgas von Brennkraftmaschinen, mit einer ein Messvolumen umgebenden Messkammer. Die Messkammer umfasst eine Zu- und Ableitung für das partikelbeladene Messgas zum bzw. vom Messvolumen. Das Streulichtmessgerät umfasst weiterhin eine Lichtquelle mit zugehöriger im Strahlweg gegenüberliegender Lichtsenke und zumindest eine im Strahlweg liegende Detektoranordnung für das von den Partikeln im Messvolumen gestreute Streulicht.

Die US 2022/120679 A1 zeigt ein selbstkalibrierendes optisches Trübungsmessgerät. Es umfasst ein Gehäuse mit einem Hohlraum. Einen optischen Standard, der zwischen einem ersten Abschnitt und einem zweiten Abschnitt innerhalb des Hohlraums beweglich ist. Eine Lichtquelle und einen Lichtsensor, die in dem ersten Abschnitt angeordnet und so konfiguriert sind, dass sie die Trübung des Mediums messen, wenn sich der optische Standard in dem zweiten Abschnitt befindet und die weiter so ausgebildet sind, um die Trübung des optischen Standards zu messen und in Abhängigkeit von der gemessenen Trübung einen Kalibrierungskoeffizienten zur Durchführung einer Selbstkalibrierung zu bestimmen, wenn sich der optische Standard im ersten Abschnitt befindet.

Die US 3 868 184 A zeigt einen optischer Teilchendetektor. Dieser umfasst ein Gehäuse, das eine Dunkelkammer umschließt und eine Lampe, die auf dem Gehäuse montiert ist. Es gibt weiterhin eine lichtempfindliche Vorrichtung, die auf das Licht reagiert, das von Partikeln gestreut wird. Weiterhin gibt es ein Partikelsimulationselement, das beweglich an dem Gehäuse angebracht ist und zur Überprüfung der Funktionsfähigkeit des Teilchendetektors dient.

Die JP 2022 138630 A beschreibt eine Branderkennungsvorrichtung, wie z.B. einen Rauchsensor zur Erkennung von Feuer.

Die CN 205 317 630 U beschreibt eine Kalibrierungseinheit für ein Partikelmessgerät.

Die US 5 751 423 A bezieht sich auf ein Partikelmessgerät, bei dem die Partikelkonzentration, die optische Transmission oder die Trübung einer gasförmigen Probe in Abhängigkeit von der Abschwächung eines durch die Probe geleiteten Lichtstrahls gemessen wird.

Es ist daher die Aufgabe der hier vorliegenden Erfindung ein Analysegerät zur Bestimmung von Feinstaub zu schaffen, welches auf einfache Weise auf seine korrekte Funktion hin überprüft werden kann

Die Aufgabe wird durch das opto-mechanische Analysegerät gemäß dem Anspruch 1 gelöst. In den Unteransprüchen sind vorteilhafte Weiterbildungen bzw. Ausführungsformen angegeben.

Das erfindungsgemäße opto-mechanische Analysegerät dient zur Bestimmung von Feinstaub in einem Messgas. Das opto-mechanische Analysegerät umfasst zumindest eine erste Lichtquelle, eine Messkammer mit einem Eingang und einem Ausgang, eine Vielzahl von Detektoren, eine Steuereinrichtung, ein optisches Referenzmesselement und eine Verschiebeeinheit mit einer Mehrgelenkmechanik. Das mit Feinstaub beladene Messgas ist der Messkammer über den Eingang zuführbar und über den Ausgang ausblasbar. Die zumindest eine erste Lichtquelle ist dazu ausgebildet, ein erstes Licht mit einer ersten Wellenlänge zu erzeugen.

Das erste Licht ist in die Messkammer einkoppelbar, wo es mit dem Messgas wechselwirken kann. Die Vielzahl der Detektoren sind an der Messkammer beabstandet voneinander angeordnet und dazu ausgebildet sind, um Streulicht zu empfangen, welches beim Auftreffen des ersten Lichts am Feinstaub entsteht. Die Detektoren sind weiter dazu ausgebildet, um Streulichtmessdaten zu erzeugen und an die Steuereinrichtung zu übertragen. Diese Übertragung kann mittels eines analogen Signals, wie beispielsweise einer analogen Spannung oder eines analogen Stroms oder mittels eines digitalen Signals erfolgen. Weiterhin ist die Verschiebeeinheit dazu ausgebildet, um das optische Referenzmesselement von einer Parkposition in eine Referenzmessposition innerhalb der Messkammer zu verschieben (d.h. zu bewegen), wobei das erste Licht auf das optische Referenzmesselement in der Referenzmessposition einstrahlbar ist und wobei die Vielzahl der Detektoren dazu ausgebildet sind, das Streulicht von dem optischen Referenzmesselement in der Referenzmessposition zu empfangen um Referenzmessdaten zu erzeugen und an die Steuereinrichtung zu übertragen. Das opto-mechanische Analysegerät weist außerdem einen Verschlussdeckel auf, der an dem optischen Referenzmesselement angeordnet ist, wobei der Verschlussdeckel in der Referenzmessposition den Eingang der Messkammer verschließt. Dadurch ist sichergestellt, dass der Messraum durch kein Messgas im Überprüfungsbetriebsmodus verunreinigt wird bzw. dass das optische Referenzmesselement nicht durch heißes Messgas beschädigt wird. Die Verschiebeeinheit mit der Mehrgelenkmechanik verschiebt das Referenzmesselement auf einer kurvenförmigen Bahn.

Erfindungsgemäß ist von Vorteil, dass ein optisches Referenzmesselement verwendet wird, welches bevorzugt mechanisch, insbesondere von außerhalb der Messkammer, in die Messkammer und damit in den Lichtstrahl des ersten Lichts eingefahren werden kann. Dadurch kann in regelmäßigen zeitlichen Abständen überprüft werden, ob sich das Ergebnis der Detektoren für die Messung des Referenzmesselements über die Zeit ändert. Ändert sich das Ergebnis der Detektoren, also ändern sich die Referenzmessdaten, so ist dies ein Hinweis darauf, dass das opto-mechanische Analysegerät eine Fehlfunktion aufweist bzw. die Messgenauigkeit stark nachgelassen hat. Denn das optische Referenzmesselement behält seine physikalischen Eigenschaften bei, weshalb eine Änderung der Referenzmessdaten über die Zeit auf eine Veränderung der Detektoren bzw. des Messaufbaus hindeutet.

Grundsätzlich kann das opto-mechanische Analysegerät in einem normalen Betriebsmodus für die eigentliche Messung von Feinstaub im Messgas betrieben werden. In diesem normalen Betriebsmodus wird die Verschiebeeinheit durch die Steuereinrichtung derart angesteuert, dass sich das optische Referenzmesselement in der Parkposition befindet. In der Parkposition befindet sich das optische Referenzmesselement bevorzugt außerhalb der Messkammer. In diesem Betriebsmodus ist das zu analysierende Messgas in die Messkammer einleitbar, wobei die Vielzahl der Detektoren dazu ausgebildet sind, um die entsprechenden Streulichtmessdaten zu erzeugen und an die Steuereinrichtung zu übertragen. Andererseits kann das opto-mechanische Analysegerät auch in einem Überprüfungsbetriebsmodus betrieben werden. In dem Überprüfungsbetriebsmodus wird die Verschiebeeinheit durch die Steuereinrichtung derart angesteuert, dass sich das optische Referenzmesselement in der Referenzmessposition befindet. In diesem Betriebsmodus wird das zumindest eine erste Licht in das optische Referenzmesselement eingekoppelt, sodass die Vielzahl der Detektoren Streulicht von dem optischen Referenzmesselement empfangen und Referenzmessdaten erzeugen und diese an die Steuereinrichtung übertragen.

In einer vorteilhaften weiteren Ausführungsform umfasst das opto-mechanische Analysegerät noch eine zweite Lichtquelle und/oder eine dritte Lichtquelle. Die zweite Lichtquelle ist dazu ausgebildet, um ein zweites Licht mit einer zweiten Wellenlänge zu erzeugen. Die dritte Lichtquelle ist dazu ausgebildet, um ein drittes Licht mit einer dritten Wellenlänge zu erzeugen. Die erste Wellenlänge, die zweite Wellenlänge und die dritte Wellenlänge sind unterschiedlich gewählt. Mittels zumindest eines entsprechendem vorzugsweise beweglichen Spiegel werden die Lichtstrahlen des ersten, zweiten und dritten Lichts auf eine gemeinsame Lichtachse gebracht, sodass alle Lichter an derselben Stelle in die Messkammer einkoppelbar sind und damit auf das optische Referenzmesselement treffen.

In einer vorteilhaften weiteren Ausführungsform liegt die Wellenlänge des ersten Lichts bei 600 bis 650 nm. Die Wellenlänge des zweiten Lichts liegt bei 900 bis 950 nm. Die Wellenlänge des dritten Lichts liegt bei 400 bis 450nm.

In einer vorteilhaften weiteren Ausführungsform ist noch eine Lichtabsorptionsvorrichtung vorhanden, die dazu ausgebildet ist, um in die Messkammer eingekoppeltes Licht zu absorbieren. Ein erster Detektor der Detektoren ist zwischen 0 ° und 6° unmittelbar neben der Lichtabsorptionsvorrichtung angeordnet und insbesondere dazu ausgebildet, um Streulicht in Vorwärtsrichtung zu erfassen. Ein zweiter Detektor ist in einem zweiten Streuwinkel bei 28° angeordnet. Ein dritter Detektor ist in einem dritten Streuwinkel bei 61 ° angeordnet. Ein vierter Detektor ist in einem vierten Streuwinkel bei 96° angeordnet. Ein fünfter Detektor ist in einem fünften Streuwinkel bei 128° angeordnet. Ein sechster Detektor ist in einem sechsten Streuwinkel bei 155° angeordnet. Dadurch können die Detektoren Streulicht in dem jeweiligen Winkel erfassen.

Gemäß der Erfindung ist die Verschiebeeinheit dazu ausgebildet, um das optische Referenzmesselement entlang einer kurvenförmigen Bewegungsbahn von der Parkposition zur Referenzmessposition zu verschieben. Vorzugsweise wird das optische Referenzmesselement nicht nur entlang einer Achse, sondern entlang von zwei Achsen bewegt.

In einer vorteilhaften weiteren Ausführungsform ist das optische Referenzmesselement in der Parkposition von dem Eingang der Messkammer weiter entfernt als in der Referenzmessposition.

In einer vorteilhaften weiteren Ausführungsform ist die Verschiebeeinheit dazu ausgebildet, um das optische Referenzmesselement zwischen der Parkposition und der Referenzmessposition zusätzlich um ungefähr oder genau 90° zu drehen, bevorzugt erfolgt die Drehung um einen Winkel von 60° bis 120°. Vorzugsweise liegt das optische Referenzmesselement in der Parkposition in der Nähe einer Seitenwand an und wird zur Einnahme der Referenzmessposition in die Messkammer hinein geklappt.

In einer vorteilhaften weiteren Ausführungsform umfasst die Verschiebeeinheit eine Mehrgelenkmechanik mit (vorzugsweise zumindest) einer Mehrgelenkverbindung, einem Antriebsmotor und einer Antriebswelle, wobei der Antriebsmotor mit der Antriebswelle drehgekoppelt ist. Dreht sich der Antriebsmotor, so dreht sich auch die Antriebswelle. Bei dem Antriebsmotor handelt es sich vorzugsweise um einen Elektromotor, insbesondere um einen Gleichstrommotor. Ein erstes Ende der Mehrgelenkverbindung ist mit der Antriebswelle drehgekoppelt. Das optische Referenzmesselement ist an einem zweiten Ende der Mehrgelenkverbindung angeordnet, insbesondere mit diesem Ende verschraubt und/oder verklebt und/oder verpresst. Eine Drehbewegung des Antriebsmotors in eine erste Richtung führt dazu, dass sich die zumindest eine Mehrgelenkverbindung ausfährt und das optische Referenzmesselement von der Parkposition in die Referenzmessposition bewegt. Wohingegen eine Drehbewegung des Antriebsmotors in eine zweite Richtung, die entgegengesetzt zur ersten Richtung ist, dazu führt, dass sich die zumindest eine Mehrgelenkverbindung einfährt und das optische Referenzmesselement von der Referenzmessposition in die Parkposition bewegt. Es ist besonders vorteilhaft, dass eine Mehrgelenkverbindung verwendet wird, weil über diese besonders effizient die kurvenförmige Bewegungsbahn für das optische Referenzmesselement realisiert werden kann.

In einer vorteilhaften weiteren Ausführungsform umfasst die Verschiebeeinheit eine Detektiereinheit, insbesondere in Form einer Positionsdetektion, z. B. einer Lichtschranke. Die Detektiereinheit ist dazu ausgebildet, um zu detektieren, ob das optische Referenzmesselement die Parkposition bzw. die Referenzmessposition erreicht hat. Die Detektiereinheit ist weiter dazu ausgebildet, um ein entsprechendes Detektiersignal an die Steuereinrichtung und/oder den Antriebsmotor auszugeben, um den Antriebsmotor zu stoppen, wenn das optische Referenzmesselement die Parkposition oder die Referenzmessposition erreicht hat.

In einer vorteilhaften weiteren Ausführungsform ist die Detektiereinheit in Form einer Positionsdetektion, z.B. einer Lichtschranke, dazu ausgebildet, um verschiedene Markierungen, die insbesondere auf der Antriebswelle angebracht sind, zu erkennen. Eine erste Markierung gibt das Erreichen der Parkposition und eine zweite Markierung gibt das Erreichen der Referenzmessposition wieder. Bei den Markierungen kann es sich beispielsweise um Vorsprünge handeln, die von der Antriebswelle in einer bestimmten Winkellage abstehen.

In einer vorteilhaften weiteren Ausführungsform ist zwischen Antriebsmotor und Antriebswelle noch ein selbsthemmendes Getriebe, insbesondere in Form eines Schneckenradgetriebes, angeordnet. Dadurch ist sichergestellt, dass das optische Referenzmesselement bei einem abgeschalteten Antriebsmotor in seiner Parkposition oder seiner Referenzmessposition verbleibt. Durch äußere Einflüsse, beispielsweise durch einen erhöhten Druck des Messgases, wird das optische Referenzmesselement nicht von seiner Referenzmessposition zurück in seine Parkposition gedrückt.

In einer vorteilhaften weiteren Ausführungsform, kommt das optische Referenzmesselement mit dem ersten Licht lediglich in der Referenzmessposition in Kontakt, nicht jedoch in der Parkposition.

In einer vorteilhaften weiteren Ausführungsform umfasst das opto-mechanische Analysegerät ein Schutzgehäuse, welches einen Aufnahmeraum umgrenzt, wobei das optische Referenzmesselement während der Parkposition in dem Aufnahmeraum angeordnet ist. Durch ein solches Schutzgehäuse ist das optische Referenzmesselement vor dem Messgas geschützt.

In einer vorteilhaften weiteren Ausführungsform ist das Schutzgehäuse außerhalb der Messkammer angeordnet.

In einer vorteilhaften weiteren Ausführungsform ist eine Überdruckeinheit vorgesehen und dazu ausgebildet, um in dem Aufnahmeraum des Schutzgehäuses einen, verglichen zum Umgebungsdruck, erhöhten Luftdruck zu erzeugen. Dadurch wird erreicht, dass kein Messgas mit Feinstaub in den Schutzraum eindringen und das optische Referenzmesselement verschmutzen kann.

In einer vorteilhaften weiteren Ausführungsform bewegt sich der Verschlussdeckel ebenfalls entlang der kurvenförmigen Bewegungsbahn, sobald sich das optische Referenzmesselement von der Parkposition in die Referenzmessposition und zurück bewegt.

In einer vorteilhaften weiteren Ausführungsform bewegt sich der Verschlussdeckel gleichzeitig mit dem optischen Referenzmesselement.

In einer vorteilhaften weiteren Ausführungsform ist ein Abstand zwischen dem Verschlussdeckel und dem optischen Referenzmesselement immer gleich und damit konstant.

In einer vorteilhaften weiteren Ausführungsform wird der Verschlussdeckel ebenfalls durch die Verschiebeeinheit bewegt.

In einer vorteilhaften weiteren Ausführungsform umfasst das Schutzgehäuse eine Öffnung, durch die das optische Referenzmesselement aus dem Aufnahmeraum herausfahrbar und in den Aufnahmeraum hineinfahrbar ist, wobei der Verschlussdeckel oder ein weiterer Verschlussdeckel vorgesehen ist, welcher die Öffnung des Schutzgehäuses in der Parkposition des optischen Referenzmesselements verschließt. Es ist besonders vorteilhaft, dass die Öffnung des Schutzgehäuses in der Parkposition des optischen Referenzmesselements verschlossen wird. Dadurch wird erreicht, dass kein Messgas in den Schutzraum eindringt und dort das optische Referenzmesselement verschmutzt. Besonders vorteilhaft ist außerdem, wenn es sich um denselben Verschlussdeckel handelt, der auch den Eingang der Messkammer in der Referenzmessposition des optischen Referenzmesselements verschließt, weil lediglich durch die kurvenförmige Bewegungsbahn des optischen Referenzmesselements und des Verschlussdeckels eine entsprechende Abdichtung, von sowohl dem Schutzraum als auch der Messkammer erreicht werden kann und keine zusätzlichen Antriebe hierfür erforderlich sind.

In einer vorteilhaften Ausführungsform umfasst der Verschlussdeckel einen runden Querschnitt.

In einer vorteilhaften Ausführungsform ist eine Reinigungseinheit vorgesehen und dazu ausgebildet, um, insbesondere gefilterte, Spülluft auf das optische Referenzmesselement in der Referenzmessposition aufzubringen. Dadurch wird verhindert, dass das optische Referenzmesselement durch das Messgas bzw. durch Rückstände des Messgases verschmutzt wird. Die Reinigungseinheit kann die Überdruckeinheit umfassen bzw. durch die Überdruckeinheit gebildet sein.

In einer vorteilhaften Ausführungsform ist ein optisches Dämpfungselement, insbesondere in Form eines Neutraldichteglases (oder auch Neutraldichtefilters), vorgesehen und dazu ausgebildet, um das erste Licht vor Eintritt in das optische Referenzmesselement in dessen Referenzmessposition zu dämpfen. Das optische Dämpfungselement ist entweder direkt am optischen Referenzmesselement angebracht oder in einem Filterrad außerhalb der Messkammer, welches durch das zumindest eine erste Licht durchstrahlt wird. Der Einsatz eines solchen optischen Dämpfungselements ist insbesondere deshalb vorteilhaft, weil das zumindest eine erste Licht an dem optischen Referenzmesselement ohne Neutraldichteglas deutlich stärker gestreut wird als an Feinstaub, der in dem Messgas enthalten ist. Durch ein solches optisches Dämpfungselement wird verhindert, dass die Detektoren zu viel Streulicht sehen und beschädigt werden bzw. lediglich den maximalen Wert für die Helligkeit ausgeben ("overload"). Das optische Dämpfungselement ist insbesondere dazu ausgebildet, um lediglich 0,1 % des zumindest einen ersten Lichts hindurchzulassen, also die Lichtleistung um ungefähr den Faktor 1000 zu verringern. Diese Dämpfung findet insbesondere im sichtbaren Spektralbereich statt.

In einer vorteilhaften Ausführungsform ist das Dämpfungselement mit dem optischen Referenzmesselement verklebt und/oder verschraubt.

In einer vorteilhaften Ausführungsform ist das optische Referenzmesselement aus einem Glas-Keramik-Medium gebildet oder umfasst ein Glas-Keramik-Medium. Bevorzugt ist das optische Referenzmesselement aus Zerodur^{®}. Zerodur^{®} ist sehr homogen und hat eine sehr niedrige Wärmeausdehnung. Dadurch ist eine reproduzierbare Bildung von Streulicht im Überprüfungsbetriebsmodus möglich.

In einer vorteilhaften Ausführungsform weist das erste Licht, bevor es in das optische Referenzmesselement in dessen Referenzmessposition eintritt, einen Strahldurchmesser von mindestens 2mm auf. Der Lichtstrahl des zumindest einen ersten Lichts kann dabei über zumindest eine entsprechende Optik, insbesondere eine Linse, aufgeweitet werden. Dadurch werden Schwankungen im Streulicht am Detektor durch etwaige Inhomogenitäten im optischen Referenzmesselement reduziert bzw. ausgeschlossen.

In einer vorteilhaften Ausführungsform ist die Steuereinrichtung dazu ausgebildet, um die Verschiebeeinheit derart anzusteuern, dass diese das optische Referenzmesselement in regelmäßigen zeitlichen Abständen von der Parkposition in die Referenzmessposition verschwenkt, wobei die Steuereinrichtung weiter dazu ausgebildet ist, um Referenzmessdaten, die zu verschiedenen Zeitpunkten erzeugt wurden, miteinander zu vergleichen und für den Fall, dass eine Abweichung oberhalb eines Schwellwerts liegt, ein Signal lokal und/oder an eine übergeordnete Steuerungsvorrichtung auszugeben. Das Signal kann lokal beispielsweise durch einen Alarm ausgegeben werden. Bei dem Alarm kann es sich um einen optischen und/oder akustischen Alarm handelt. Die Steuereinrichtung vergleicht vorzugsweise Referenzmessdaten, die an verschiedenen Tagen oder Monaten aufgenommen wurden miteinander. Liegt eine Abweichung oberhalb eines Schwellwerts vor, so kann ein Techniker eine Überprüfung des opto-mechanischen Analysegeräts vornehmen.

In einer vorteilhaften Ausführungsform umfasst ein Leitungssystem, welches das mit Feinstaub beladene Messgas in Richtung des Eingangs der Messkammer transportiert, eine Verjüngung im Querschnitt, insbesondere im Durchmesser. Die Verjüngung erstreckt sich lediglich über eine bestimmte Länge. Vor und nach der Verjüngung ist der Querschnitt wieder größer als im Bereich der Verjüngung.

Nachfolgend wird die Erfindung rein beispielhaft unter Bezugnahme auf die Zeichnungen beschrieben. Es zeigen:
- Figur 1:: ein Ausführungsbeispiel eines erfindungsgemäßen opto-mechanischen Analysegeräts, in dessen Messkammer ein mit Feinstaub beladenes Messgas eingeleitet wird;
- Figur 2:: ein Ausführungsbeispiel des erfindungsgemäßen opto-mechanischen Analysegeräts, in dessen Messkammer das optische Referenzmesselement eingeschwenkt ist;
- Figur 3:: ein Ausführungsbeispiel des erfindungsgemäßen opto-mechanischen Analysegeräts, welches die Dämpfung eines Lichts beschreibt, welches in das optische Referenzmesselement einkoppelt;
- Figur 4:: verschiedene Darstellungen des optischen Referenzmesselements;
- Figur 5:: einen beispielhaften Verlauf von Referenzmessdaten, die durch die verschiedenen Detektoren über einen bestimmten Zeitraum aufgenommen wurden;
- Figur 6:: einen Schnitt durch das erfindungsgemäße opto-mechanische Analysegerät, welches den Weg des Messgases und die Lage des optischen Referenzmesselement beschreibt;
- Figur 7:: eine räumliche Darstellung eines Teils des opto-mechanischen Analysegeräts;
- Figur 8:: eine räumliche Darstellung eines Teils des opto-mechanischen Analysegeräts; und
- Figur 9:: eine räumliche Darstellung eines Teils des opto-mechanischen Analysegeräts, um die Verschiebeeinheit zu beschreiben.

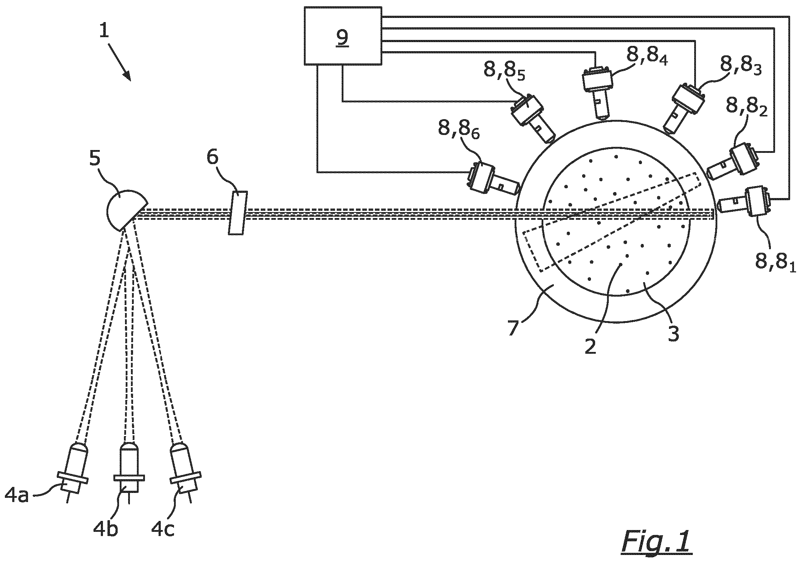

Figur 1 zeigt ein Ausführungsbeispiel eines opto-mechanischen Analysegeräts 1, welches zur Bestimmung von Feinstaub 2 in einem Messgas 3 dient. Das opto-mechanische Analysegerät 1 umfasst in diesem Fall eine erste Lichtquelle 4a, eine zweite Lichtquelle 4b und eine dritte Lichtquelle 4c. Die erste Lichtquelle 4a ist dazu ausgebildet, um Licht mit einer ersten Wellenlänge zu erzeugen und auszusenden. Die zweite Lichtquelle 4b ist dazu ausgebildet, um Licht mit einer zweiten Wellenlänge zu erzeugen und auszusenden. Die dritte Lichtquelle 4c ist dazu ausgebildet, um Licht mit einer dritten Wellenlänge zu erzeugen und auszusenden. Über einen entsprechenden Spiegel 5 werden die Lichtstrahlen der ersten, zweiten und dritten Lichtquelle 4a, 4b, 4c zu einem gemeinsamen Lichtstrahl gebündelt. Über eine Optik 6 kann der zumindest eine Lichtstrahl oder die mehreren Lichtstrahlen auf eine bestimmte Weite aufgeweitet werden. Insbesondere hat jeder Lichtstrahl eine Weite von vorzugsweise mehr als 2 mm. Die Optik 6 kann auch direkt in der ersten, zweiten und dritten Lichtquelle 4a, 4b, 4c integriert sein.

Das opto-mechanische Analysegerät 1 umfasst außerdem eine Messkammer 7, die einen Eingang 7a und einen Ausgang 7b aufweist. Über den Eingang 7a ist das Messgas 3 der Messkammer 7 zuführbar und über den Ausgang 7b ist das Messgas 3 aus der Messkammer 7 abführbar.

Weiterhin sind noch eine Vielzahl von Detektoren 8 vorgesehen, die an der Messkammer 7 beabstandet voneinander, also in einem gewissen Winkelabstand zueinander angeordnet und dazu ausgebildet sind, um Streulicht zu empfangen, welches beim Auftreffen des Lichts am Feinstaub 2 entsteht.

Die Messkammer 7 umfasst oder besteht vorzugsweise aus Metall. Die Messkammer 7 weist verschiedene Öffnungen auf. In diesen Öffnungen sind vorzugsweise die Detektoren 8 angeordnet. Spalte an den Öffnungen sind bevorzugt luftdicht abgeschlossen. Eine weitere Eintrittsöffnung dient zum Einkoppeln des Lichts der zumindest einen ersten Lichtquelle 4a. Vorzugsweise gibt es auch eine Austrittsöffnung, über die das eingekoppelte Licht wieder aus der Messkammer 7 herausgeführt und insbesondere in einen optischen Sumpf abgeleitet wird. Ein solcher optischer Sumpf hat die Eigenschaft, dass so gut wie keine Lichtanteile mehr reflektiert werden. Der optische Sumpf kann auch als Lichtfalle bezeichnet werden.

Der optische Sumpf kann auch als Lichtabsorptionsvorrichtung bezeichnet werden. Die Lichtabsorptionsvorrichtung ist bei 0° angeordnet. Ein erster Detektor 8₁ der Detektoren 8 ist zwischen 0° und 6° unmittelbar neben der Lichtabsorptionsvorrichtung angeordnet und insbesondere dazu ausgebildet, um Streulicht in Vorwärtsrichtung zu erfassen. Ein zweiter Detektor 8₂ ist in einem zweiten Streuwinkel bei 28° angeordnet. Ein dritter Detektor 8₃ ist in einem dritten Streuwinkel bei 61° angeordnet. Ein vierter Detektor 8₄ ist in einem vierten Streuwinkel bei 96° angeordnet. Ein fünfter Detektor 8₅ ist in einem fünften Streuwinkel bei 128° angeordnet. Ein sechster Detektor 8₆ ist in einem sechsten Streuwinkel bei 155° angeordnet. Dadurch können die Detektoren 8 Streulicht in dem jeweiligen Winkel erfassen.

Die Messkammer 7 hat vorzugsweise einen runden Querschnitt und ist weiter vorzugsweise als Hohlzylinder ausgeführt. Dabei umgrenzt die Messkammer 7 einen Gasaufnahmeraum, in den das Messgas 3 eingeleitet wird.

Die Detektoren 8 sind vorzugsweise an der Außenwandung der Messkammer 7 angeordnet. Die Detektoren 18 sind vorzugsweise in derselben Ebene angeordnet. In dieser Ebene verläuft vorzugsweise auch der Lichtstrahl der zumindest einen ersten Lichtquelle 4a.

Weiterhin umfasst das opto-mechanische Analysegerät 1 noch eine Steuereinrichtung 9. Die Detektoren 8 sind dazu ausgebildet, um Streulicht zu empfangen und in Abhängigkeit der Helligkeit Streulichtmessdaten zu erzeugen und diese Streulichtmessdaten an die Steuereinrichtung 9 zu übertragen. Dies alles erfolgt in einem normalen Betriebsmodus des opto-mechanischen Analysegeräts 1. Die Steuereinrichtung 9 kann auch dazu ausgebildet sein, um die Streulichtmessdaten an eine übergeordnete Steuerungsvorrichtung, die auch als Leiteinrichtung bezeichnet werden kann, zu übertragen.

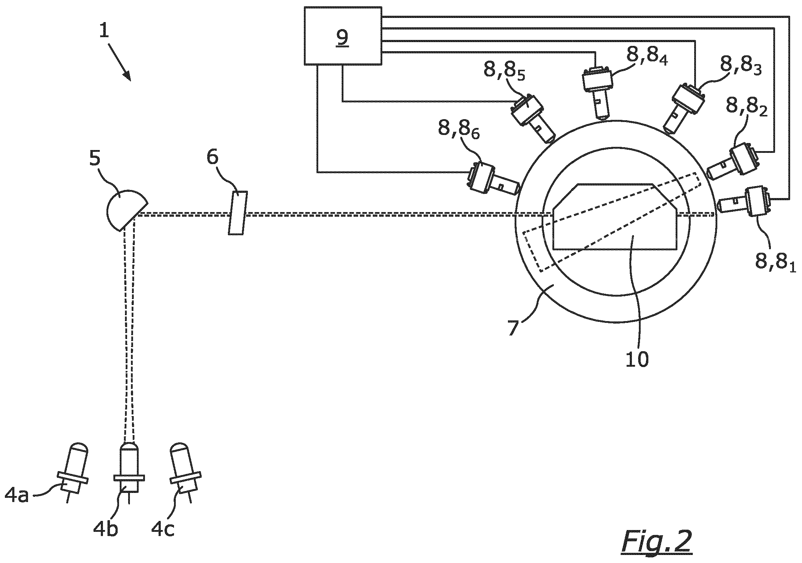

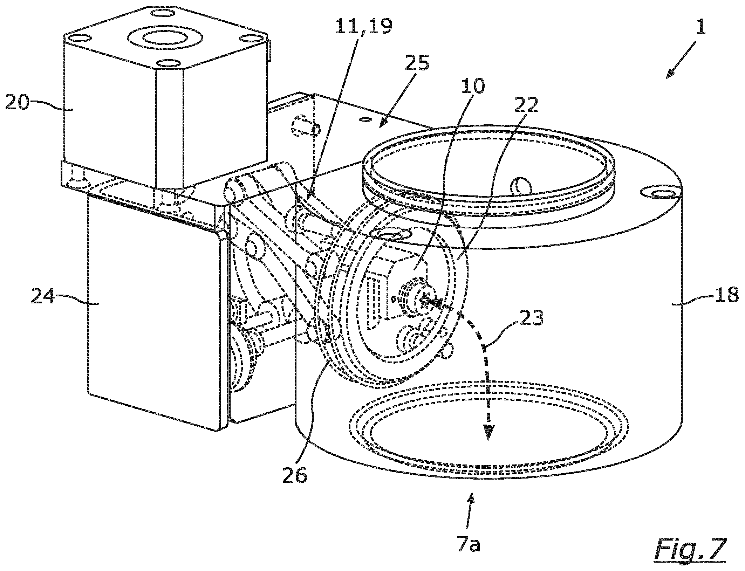

Figur 2 zeigt ein weiteres Ausführungsbeispiel des erfindungsgemäßen opto-mechanischen Analysegeräts 1. Das opto-mechanische Analysegerät 1 umfasst ein optisches Referenzmesselement 10 und eine Verschiebeeinheit 11 (siehe Figuren 7, 8, 9). Die Verschiebeeinheit 11 ist dazu ausgebildet, um das optische Referenzmesselement 10 von einer Parkposition in eine Referenzmessposition zu verschieben, wobei das erste Licht in das optische Referenzmesselement 10 in der Referenzmessposition einstrahlbar ist und wobei die Vielzahl der Detektoren 8 dazu ausgebildet sind, um Streulicht von dem optischen Referenzmesselement 10 in der Referenzmessposition zu empfangen und Referenzmessdaten zu erzeugen und an die Steuereinrichtung 9 zu übertragen. Figur 2 zeigt das optische Referenzmesselement 10 in seiner Referenzmessposition, wohingegen sich das optische Referenzmesselement 10 in Figur 1 in seiner Parkposition befindet und daher nicht dargestellt ist.

Das optische Referenzmesselement 10 ist aus einem Glas-Keramik-Medium gebildet oder umfasst ein Glas-Keramik-Medium und ist insbesondere Zerodur^{®}.

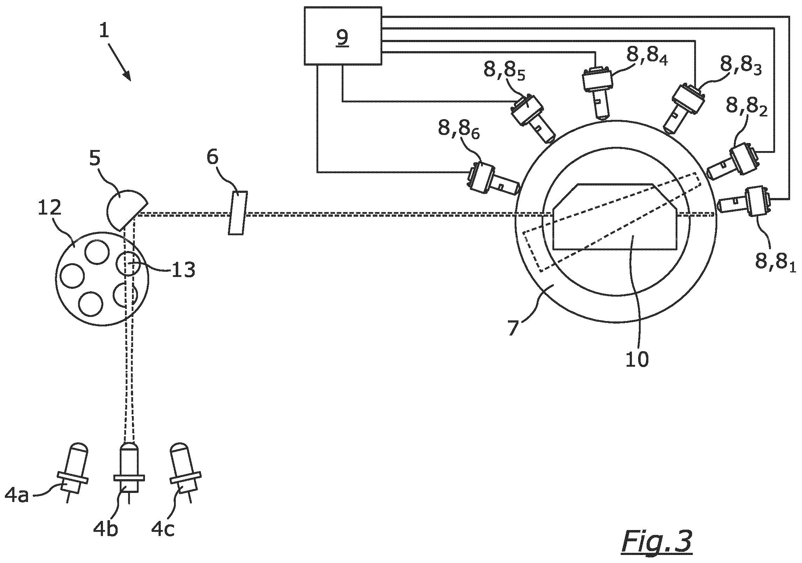

Figur 3 zeigt ein weiteres Ausführungsbeispiel des opto-mechanischen Analysegeräts 1. Es wird erläutert, dass noch ein optisches Dämpfungselement 12 vorgesehen und dazu ausgebildet ist, um das erste Licht vor Eintritt in das optische Referenzmesselement 10 in dessen Referenzmessposition zu dämpfen, wobei das optische Dämpfungselement 12 in einem Filterrad 13 außerhalb der Messkammer 7 angeordnet ist, welches durch das erste Licht durchsetzt wird. Dadurch wird dem Umstand Rechnung getragen, dass in dem optischen Referenzmesselement 10 im Überprüfungsbetriebsmodus mehr Licht gestreut wird als im normalen Betriebsmodus in einem mit Feinstaub 2 beladenem Messgas 3. Die Detektoren 8 übermitteln daher nicht den größtmöglichen Helligkeitswert an die Steuereinrichtung 9 bzw. werden nicht beschädigt.

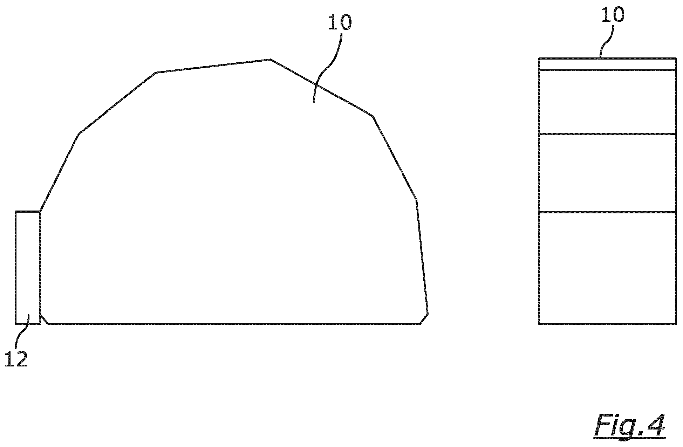

Figur 4 zeigt ein Ausführungsbeispiel des optischen Referenzmesselements 10. Die Darstellung auf der linken Seite zeigt einen Blick auf die Ober- oder Unterseite des optischen Referenzmesselements 10. Das optische Referenzmesselement 10 ist ein n-Eck mit n ≥ 5. Vorzugweise umfasst das optische Referenzmesselement 10 so viele Ecken bzw. abgewinkelte Kanten wie es Detektoren 8 gibt. Die Darstellung auf der rechten Seite zeigt ein Blick auf eine Seite des optischen Referenzmesselements 10. Das optische Referenzmesselement 10 ist länger als es dick ist. Dargestellt ist außerdem, dass das optische Dämpfungselement 12 mit einer Seite des optischen Referenzmesselements 10 verbunden, insbesondere verklebt ist. Über das optische Dämpfungselement 12 wird zumindest das eine erste Licht der ersten Lichtquelle 4a in das optische Referenzmesselement 10 eingestrahlt.

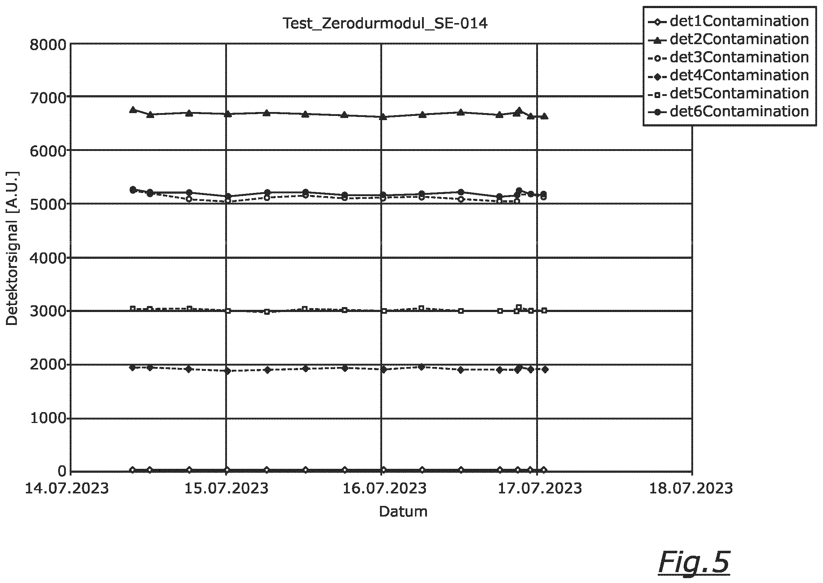

Figur 5 zeigt eine Vielzahl von Referenzmessdaten, die zu unterschiedlichen Zeitpunkten von unterschiedlichen Detektoren 8 aufgenommen wurden. Auf der Abszisse ist die Zeit aufgetragen und auf der Koordinate die Detektorleistung. Die verschiedenen Messwertkurven mit Referenzmessdaten sind durch die unterschiedlichen Detektoren 8 erzeugt. So ist die Verschiebeeinheit 11 dazu ausgebildet, um das optische Referenzmesselement 10 in regelmäßigen zeitlichen Abständen von der Parkposition in die Referenzmessposition zu verschieben. In der Referenzmessposition wird das Licht der zumindest einen ersten Lichtquelle 4a in das optische Referenzmesselement 10 eingekoppelt, weshalb die Vielzahl der Detektoren 8 dazu ausgebildet sind, um Streulicht von dem optischen Referenzmesselement 10 zu messen und entsprechende Referenzmessdaten zu erzeugen. Zu erkennen ist, dass es in den Referenzmessdaten einen Sprung gibt, der auf eine Anomalie in dem opto-mechanischen Analysegerät 1 hindeutet.

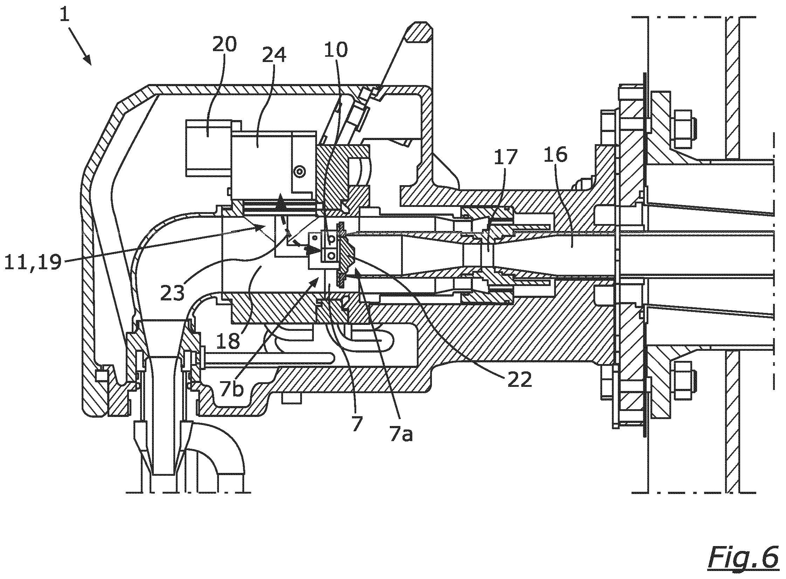

Figur 6 zeigt einen Schnitt durch das erfindungsgemäße opto-mechanische Analysegerät 1, welches den Weg des Messgases 3 beschreibt und die Lage des optischen Referenzmesselements 10. Das mit Feinstaub 2 beladene Messgas 3 wird über eine Probenahmeleitung 16 der Messkammer 7 zugeführt. Die Probenahmeleitung 16 umfasst eine Verjüngung 17 im Querschnitt. Diese Verjüngung 17 erstreckt sich lediglich über eine bestimmte Länge, sodass vor und nach dieser Verjüngung 17 der Querschnitt größer ist als im Bereich der Verjüngung 17. Am Ausgang 7b der Messkammer 7 schließt sich ein Messrohr 18 an.

In Figur 6 befindet sich das optische Referenzmesselement 10 in der Messkammer 7 und damit in seiner Referenzmessposition. Das optische Referenzmesselement 10 ist mit der Mehrgelenkmechanik 19 verbunden.

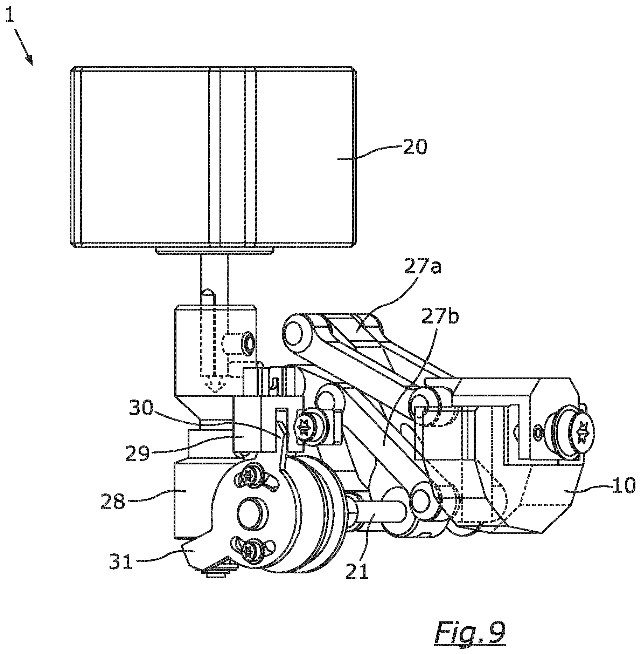

Die Verschiebeeinheit 11 umfasst eine Mehrgelenkmechanik 19, einen Antriebsmotor 20 und eine Antriebswelle 21 (siehe Figur 9). Weiterhin ist ein Verschlussdeckel 22 vorgesehen, der an der Mehrgelenkmechanik 19 und/oder an dem optischen Referenzmesselement 10 angeordnet ist und in der Referenzmessposition, die in Figur 6 dargestellt ist, den Eingang 7a der Messkammer 7, insbesondere nahezu luftdicht verschließt. Dadurch wird verhindert, dass mit Feinstaub 2 beladenes Messgas 3 im Überprüfungsbetriebsmodus in die Messkammer 7 einströmt und insbesondere das optische Referenzmesselement 10 verschmutzt.

Die Verschiebeeinheit 11 ist dazu ausgebildet, um das optische Referenzmesselement 10 entlang einer kurvenförmigen Bewegungsbahn 23 von der Parkposition zur Referenzmessposition und zurück zu verschieben. Die kurvenförmige Bewegungsbahn 23 ist in Figur 6 gestrichelt dargestellt. Die Verschiebeeinheit 11 ist dazu ausgebildet, um das optische Referenzmesselement 10 zwischen der Parkposition und der Referenzmessposition um ungefähr 90° zu drehen.

In Figur 6 ist weiterhin ein Schutzgehäuse 24 dargestellt, welches einen Aufnahmeraum 25 (siehe Figur 7) umgrenzt. In der Parkposition befindet sich das optische Referenzmesselement 10 im Aufnahmeraum 25 innerhalb des Schutzgehäuses 24. Dieser Sachverhalt ist in Figur 7 dargestellt.

Figur 7 zeigt eine vergrößerte Darstellung eines Teils des opto-mechanischen Analysegeräts 1. Die Messkammer 7 ist hier weggelassen. Vom Aufnahmeraum 25 des Schutzgehäuse 24 wird das optische Referenzmesselement 10 entlang der Bewegungsbahn 23, in diesem Fall, durch das Messrohr 18 in Richtung des Eingangs 7a der Messkammer 7 verschoben. Zeitgleich wird auch der Verschlussdeckel 22 in Richtung des Eingangs 7a der Messkammer 7 verschoben. Der Verschlussdeckel 22 umfasst zumindest eine, vorzugsweise zwei umlaufende Dichtungen 26. In der Referenzmessposition ist der Verschlussdeckel 22 dazu ausgebildet, um den Eingang 7a der Messkammer 7, vorzugsweise luftdicht, zu verschließen. In der Parkposition ist derselbe Verschlussdeckel 22 dazu ausgebildet, um eine Öffnung in dem Schutzgehäuse 24, durch welche das optische Referenzmesselement 10 vom Aufnahmeraum 25, insbesondere in das Messrohr 18, ausgefahren werden kann, zu verschließen. Insbesondere verschließt der Verschlussdeckel 22 diese Öffnung ebenfalls nahezu luftdicht.

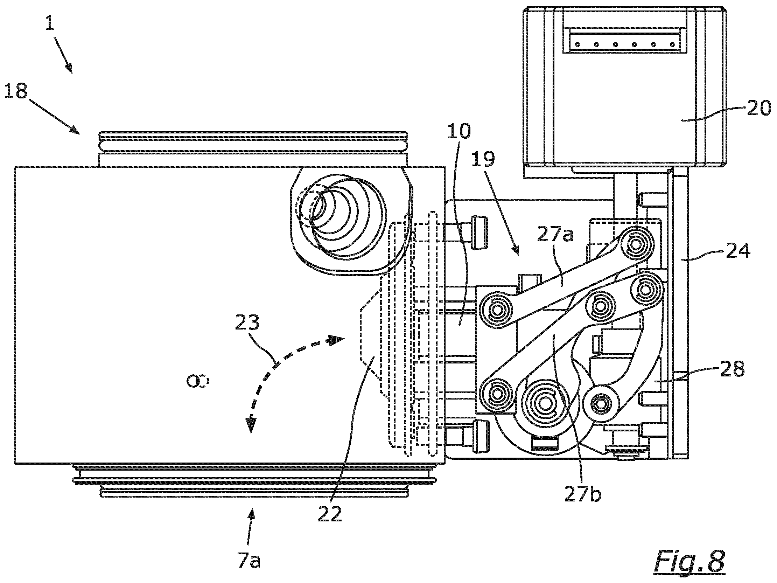

Die Figuren 8 und 9 zeigen eine weitere Darstellung des opto-mechanischen Analysegeräts 1 und erläutern insbesondere die Wirkungsweise der Verschiebeeinheit 11 und dabei insbesondere der Mehrgelenkmechanik 19. Die Mehrgelenkmechanik 19 ist in diesem Fall eingefahren, was bedeutet, dass sich das optische Referenzmesselement 10 in der Parkposition befindet.

Der Antriebsmotor 20 ist über ein Getriebe 28 mit der Antriebswelle 21 verbunden. Bei dem Getriebe 28 handelt es sich vorzugsweise um ein selbsthemmendes Getriebe .

Die Mehrgelenkmechanik 19 umfasst eine erste Mehrgelenkverbindung 27a und eine zweite Mehrgelenkverbindung 27b. Die erste Mehrgelenkverbindung 27a ist mit einem ersten Ende mit der Antriebswelle 21 drehgekoppelt. Dreht sich die Antriebswelle 21, so dreht sich auch das erste Ende der ersten Mehrgelenkverbindung 27a. Das zweite Ende der ersten Mehrgelenkverbindung 27a ist mit dem optischen Referenzmesselement 10 verbunden. Die zweite Mehrgelenkverbindung 27b kann mit ihrem ersten Ende mit der Antriebswelle 21 drehgekoppelt sein oder drehbar an einem stationären Teil der Mehrgelenkmechanik 19 bzw. des Schutzgehäuses 24 angeordnet sein. In diesem Fall würde die Kraft zum Ausfahren der Mehrgelenkmechanik 19 einzig über die erste Mehrgelenkverbindung 27a übertragen werden. Ein zweites Ende der zweiten Mehrgelenkverbindung 27b ist wiederum mit dem optischen Referenzmesselement 10 verbunden. Das optische Referenzmesselement 10 kann über eine Schraubverbindung und/oder Klemmverbindung und/oder Klebeverbindung mit der Mehrgelenkmechanik 19 verbunden sein.

Sowohl die erste Mehrgelenkverbindung 27a als auch die zweite Mehrgelenkverbindung 27b umfassen mehrere Arme, die über ein Gelenk (in Reihe) miteinander verbunden sind.

Insbesondere über die Verbindung der ersten Mehrgelenkverbindung 27a mit der Antriebsachse 21 und im Zusammenspiel mit den weiteren Drehpunkten der gesamten Mehrgelenkmechanik ist es möglich, dass das optische Referenzmesselement 10 einer kurvenförmigen Bewegungsbahn 23 folgt. Bevorzugt greifen das zweite Ende der ersten Mehrgelenkverbindung 27a und das zweite Ende der zweiten Mehrgelenkverbindung 27b an unterschiedlichen Stellen am optischen Referenzmesselement 10 an.

In Figur 9 ist der Verschlussdeckel 22 nicht dargestellt.

Die Verschiebeeinheit 11 umfasst weiterhin eine Detektiereinheit 29, insbesondere in Form einer Lichtschranke, die dazu ausgebildet ist, um zu detektieren, ob das optische Referenzmesselement 10 die Parkposition oder Referenzmessposition erreicht hat. Die Detektiereinheit 29 ist dazu ausgebildet ist, um ein entsprechendes Detektiersignal an die Steuereinrichtung 9 und/oder den Antriebsmotor 20 auszugeben, um den Antriebsmotor 20 zu stoppen.

Die Detektiereinheit 29 in Form der Lichtschranke ist dazu ausgebildet, um verschiedene Markierungen 30, 31, die insbesondere auf der Antriebswelle 21 angebracht sind, zu erkennen. Eine erste Markierung 30 gibt das Erreichen der Parkposition und eine zweite Markierung 31 gibt das Erreichen der Referenzmessposition wieder. Bei den Markierungen 30, 31 kann es sich beispielsweise um Vorsprünge handeln, die von der Antriebswelle 21 in einer bestimmten Winkellage abstehen.

**Bezugszeichenliste**

| | |
|---|---|
| Opto-mechanisches Analysegerät | 1 |
| Feinstaub | 2 |
| Messgas | 3 |
| Erste Lichtquelle | 4a |
| Zweite Lichtquelle | 4b |
| Dritte Lichtquelle | 4c |
| Spiegel | 5 |
| Optik | 6 |
| Messkammer | 7 |
| Eingang (Messkammer) | 7a |
| Ausgang (Messkammer) | 7b |
| Detektoren | 8, 8₁, 8₂, 8₃, 8₄, 8₅, 8₆ |
| Steuereinrichtung | 9 |
| Optisches Referenzmesselement | 10 |
| Verschiebeeinheit | 11 |
| Optisches Dämpfungselement | 12 |
| Filterrad | 13 |
| Probenahmeleitung | 16 |
| Verjüngung | 17 |
| Messrohr | 18 |
| Mehrgelenkmechanik | 19 |
| Antriebsmotor | 20 |
| Antriebswelle | 21 |
| Verschlussdeckel | 22 |
| Kurvenförmige Bewegungsbahn | 23 |
| Schutzgehäuse | 24 |
| Aufnahmeraum | 25 |
| Umlaufende Dichtung(en) | 26 |
| Erste Mehrgelenkverbindung | 27a |
| Zweite Mehrgelenkverbindung | 27b |
| Getriebe | 28 |
| Detektiereinheit | 29 |
| Erste Markierung | 30 |
| Zweite Markierung | 31 |

## Patentansprüche

1. Opto-mechanisches Analysegerät (1) zur Bestimmung von Feinstaub (2) in einem Messgas (3), wobei das opto-mechanische Analysegerät (1) zumindest eine erste Lichtquelle (4a), eine Messkammer (7) mit einem Eingang (7a) und einem Ausgang (7b), eine Vielzahl von Detektoren (8), eine Steuereinrichtung (9), ein optisches Referenzmesselement (10) und eine Verschiebeeinheit (11) umfasst, wobei
das mit Feinstaub (2) beladene Messgas (3) der Messkammer (7) über den Eingang (7a) zuführbar und über den Ausgang (7b) auslassbar ist, wobei die zumindest eine erste Lichtquelle (4a) dazu ausgebildet ist, um ein erstes Licht mit einer ersten Wellenlänge zu erzeugen, wobei das erste Licht in die Messkammer (7) einkoppelbar ist, wobei
die Vielzahl der Detektoren (8) an der Messkammer (7) beabstandet voneinander angeordnet und dazu ausgebildet sind, Streulicht zu empfangen, welches beim Auftreffen des ersten Lichts am Feinstaub (2) entsteht, und Streulichtmessdaten zu erzeugen und an die Steuereinrichtung (9) zu übertragen, wobei die Verschiebeeinheit (11) dazu ausgebildet ist, um das optische Referenzmesselement (10) von einer Parkposition in eine Referenzmessposition zu verschieben, wobei das erste Licht in das optische Referenzmesselement (10) in der Referenzmessposition einstrahlbar ist und wobei die Vielzahl der Detektoren (8) dazu ausgebildet sind, um Streulicht von dem optischen Referenzmesselement (10) in der Referenzmessposition zu empfangen und Referenzmessdaten zu erzeugen und an die Steuereinrichtung (9) zu übertragen,
wobei das opto-mechanische Analysegerät (1) einen Verschlussdeckel (22) aufweist, der an dem optischen Referenzmesselement (10) angeordnet ist,
wobei der Verschlussdeckel (22) in der Referenzmessposition den Eingang (7a) der Messkammer (7) verschließt, **dadurch gekennzeichnet, dass** die Verschiebeeinheit (11) eine Mehrgelenkmechanik aufweist und selement (10) auf einer kurvenförmigen Bahn verschiebt.

2. Opto-mechanisches Analysegerät (1) nach Anspruch 1, wobei die Verschiebeeinheit (11) dazu ausgebildet ist, um das optische Referenzmesselement (10) zwischen der Parkposition und der Referenzmessposition zwischen 60° und 120° und insbesondere um 90° zu drehen.

3. Opto-mechanisches Analysegerät (1) nach einem der vorstehenden Ansprüche, wobei die Verschiebeeinheit (11) eine Mehrgelenkmechanik (19) mit zumindest einer Mehrgelenkverbindung (27a, 27b), einen Antriebsmotor (20) und eine Antriebswelle (21) umfasst, wobei der Antriebsmotor (20) mit der Antriebswelle (21) drehgekoppelt ist, wobei
ein erstes Ende der zumindest einen Mehrgelenkverbindung (27a, 27b) mit der Antriebswelle (21) drehgekoppelt ist und wobei das optische Referenzmesselement (20) an einem zweiten Ende der zumindest einen Mehrgelenkverbindung (27a, 27b) angeordnet ist, wobei eine Drehbewegung des Antriebsmotors (20) in eine erste Richtung dazu führt, dass sich die zumindest eine Mehrgelenkverbindung (27a, 27b) ausfährt und das optische Referenzmesselement (10) von der Parkposition in die Referenzmessposition bewegt und wobei eine Drehbewegung des Antriebsmotors (20) in eine zweite Richtung, die entgegengesetzt zur ersten Richtung ist, dazu führt, dass sich die zumindest eine Mehrgelenkverbindung (27a, 27b) einfährt und das optische Referenzmesselement (10) von der Referenzmessposition in die Parkposition bewegt.

4. Opto-mechanisches Analysegerät (1) nach Anspruch 3, wobei die Verschiebeeinheit (11) eine Detektiereinheit (29), insbesondere in Form einer Lichtschranke, umfasst, die dazu ausgebildet ist, um zu detektieren, ob das optische Referenzmesselement (10) die Parkposition oder Referenzmessposition erreicht hat, wobei die Detektiereinheit (29) dazu ausgebildet ist, um ein entsprechendes Detektiersignal an die Steuereinrichtung (9) und/oder den Antriebsmotor (20) auszugeben, um den Antriebsmotor (20) zu stoppen.

5. Opto-mechanisches Analysegerät (1) nach Anspruch 3 oder 4, wobei zwischen Antriebsmotor (20) und Antriebswelle (21) noch ein selbsthemmendes Getriebe (29), insbesondere in Form eines Schneckenradgetriebes, angeordnet ist, wodurch das optische Referenzmesselement (10) bei einem abgeschalteten Antriebsmotor (20) in seiner Parkposition oder seiner Referenzmessposition verbleibt.

6. Opto-mechanisches Analysegerät (1) nach einem der vorstehenden Ansprüche, wobei das opto-mechanische Analysegerät (1) ein Schutzgehäuse (24) umfasst, welches einen Aufnahmeraum (25) umgrenzt, wobei in dem Aufnahmeraum (25) das optische Referenzmesselement (10) während der Parkposition angeordnet ist.

7. Opto-mechanisches Analysegerät (1) nach Anspruch 4, wobei eine Überdruckeinheit vorgesehen und dazu ausgebildet ist, um in dem Aufnahmeraum (25) des Schutzgehäuse (24) einen, verglichen zum Umgebungsdruck, erhöhten Luftdruck zu erzeugen.

8. Opto-mechanisches Analysegerät (1) nach einem der Ansprüche 6 oder 7, wobei das Schutzgehäuse (24) eine Öffnung umfasst, durch die das optische Referenzmesselement (10) aus dem Aufnahmeraum (25) herausfahrbar und in den Aufnahmeraum (25) hineinfahrbar ist, wobei der Verschlussdeckel (22) die Öffnung des Schutzgehäuses (24) in der Parkposition verschließt.

9. Opto-mechanisches Analysegerät (1) nach einem der vorstehenden Ansprüche, wobei eine Reinigungseinheit vorgesehen und dazu ausgebildet ist, um, insbesondere gefilterte, Spülluft in die Messkammer (7) einzublasen, wenn sich das optische Referenzmesselement (10) in der Referenzmessposition befindet.

10. Opto-mechanisches Analysegerät (1) nach einem der vorstehenden Ansprüche, wobei ein optisches Dämpfungselement (12), insbesondere in Form eines Neutraldichteglases, vorgesehen und dazu ausgebildet ist, um das erste Licht vor Eintritt in das optische Referenzmesselement (10) in dessen Referenzmessposition zu dämpfen, wobei das optische Dämpfungselement (12) direkt am optischen Referenzmesselement (10) angebracht ist oder in einem Filterrad (13) außerhalb der Messkammer (7), welches durch das erste Licht durchstrahlt wird.

11. Opto-mechanisches Analysegerät (1) nach einem der vorstehenden Ansprüche, wobei das optische Referenzmesselement (10) aus einem Glas-Keramik-Medium gebildet ist oder ein Glas-Keramik-Medium umfasst und insbesondere Zerodur^{®} ist.

12. Opto-mechanisches Analysegerät (1) nach einem der vorstehenden Ansprüche, wobei das erste Licht bevor es in das optische Referenzmesselement (10) in dessen Referenzmessposition eintritt, einen Strahldurchmesser von mindestens 2mm aufweist.

13. Opto-mechanisches Analysegerät (1) nach einem der vorstehenden Ansprüche, wobei die Steuereinrichtung (9) dazu ausgebildet ist, um die Verschiebeeinheit (11) derart anzusteuern, dass diese das optische Referenzmesselement (10) in regelmäßigen Abständen von der Parkposition in die Referenzmessposition verschwenkt, wobei die Steuereinrichtung (9) weiter dazu ausgebildet ist, um Referenzmessdaten, die zu verschiedenen Zeitpunkten erzeugt wurden, miteinander zu vergleichen und für den Fall, dass eine Abweichung oberhalb eines Schwellwerts liegt, ein Signal lokal und/oder an eine übergeordnete Steuerungsvorrichtung auszugeben.

## Claims

1. An opto-mechanical analysis device (1) for determining particulate matter (2) in a measurement gas (3), wherein the opto-mechanical analysis device (1) comprises at least one first light source (4a), a measurement chamber (7) having an inlet (7a) and an outlet (7b), a plurality of detectors (8), a control device (9), an optical reference measurement element (10) and a displacement unit (11), wherein
the measurement gas (3) loaded with particulate matter (2) can be supplied to the measurement chamber (7) via the inlet (7a) and can be discharged via the outlet (7b), wherein
the at least one first light source (4a) is configured to generate a first light having a first wavelength, wherein the first light can be coupled into the measurement chamber (7), wherein
the plurality of detectors (8) are arranged spaced apart from one another at the measurement chamber (7) and are configured to receive scattered light, which is produced on the incidence of the first light on the particulate matter (2), and to generate scattered light measurement data and to transmit said data to the control device (9), wherein the displacement unit (11) is configured to displace the optical reference measurement element (10) from a parking position into a reference measurement position, wherein the first light can be radiated into the optical reference measurement element (10) in the reference measurement position, and wherein the plurality of detectors (8) are configured to receive scattered light from the optical reference measurement element (10) in the reference measurement position and to generate reference measurement data and to transmit said data to the control device (9),
wherein the opto-mechanical analysis device (1) comprises a closing cover (22) which is arranged at the optical reference measurement element (10), wherein the closing cover (22) closes the inlet (7a) of the measurement chamber (7) in the reference measurement position, **characterized in that** the displacement unit (11) has a multi-joint mechanism and displaces the reference measurement element (10) on a curved path.

2. An opto-mechanical analysis device (1) according to claim 1, wherein the displacement unit (11) is configured to rotate the optical reference measurement element (10) between the parking position and the reference measurement position by between 60° and 120° and in particular by 90°.

3. An opto-mechanical analysis device (1) according to one of the preceding claims, wherein the displacement unit (11) comprises a multi-joint mechanism (19) having at least one multi-joint connection (27a, 27b), a drive motor (20) and a drive shaft (21), wherein the drive motor (20) is rotationally coupled to the drive shaft (21), wherein
a first end of the at least one multi-joint connection (27a, 27b) is rotationally coupled to the drive shaft (21) and wherein the optical reference measurement element (20) is arranged at a second end of the at least one multi-joint connection (27a, 27b), wherein a rotary movement of the drive motor (20) in a first direction causes the at least one multi-joint connection (27a, 27b) to extend and the optical reference measurement element (10) to move from the parking position into the reference measurement position, and wherein a rotary movement of the drive motor (20) in a second direction, which is opposite the first direction, causes the at least one multi-joint connection (27a, 27b) to retract and the optical reference measurement element (10) to move from the reference measurement position into the parking position.

4. An opto-mechanical analysis device (1) according to claim 3, wherein the displacement unit (11) comprises a detection unit (29), in particular in the form of a light barrier, which is configured to detect whether the optical reference measurement element (10) has reached the parking position or the reference measurement position, wherein the detection unit (29) is configured to output a corresponding detection signal to the control device (9) and/or the drive motor (20) in order to stop the drive motor (20).

5. An opto-mechanical analysis device (1) according to claim 3 or 4, wherein a self-locking gear (29), in particular in the form of a worm gear, is also arranged between the drive motor (20) and the drive shaft (21), whereby the optical reference measurement element (10) remains in its parking position or its reference measurement position when a drive motor (20) is switched off.

6. An opto-mechanical analysis device (1) according to any one of the preceding claims, wherein the opto-mechanical analysis device (1) comprises a protective housing (24) which defines a receiving space (25), wherein the optical reference measurement element (10) is arranged in the receiving space (25) during the parking position.

7. An opto-mechanical analysis device (1) according to claim 4, wherein an overpressure unit is provided and is configured to generate an increased air pressure, compared to the environmental pressure, in the receiving space (25) of the protective housing (24).

8. An opto-mechanical analysis device (1) according to one of the claims 6 or 7, wherein the protective housing (24) comprises an opening through which the optical reference measurement element (10) can be moved out of the receiving space (25) and into the receiving space (25), wherein the closing cover (22) closes the opening of the protective housing (24) in the parking position.

9. An opto-mechanical analysis device (1) according to any one of the preceding claims, wherein a cleaning unit is provided and is configured to blow purge air, in particular filtered purge air, into the measurement chamber (7) when the optical reference measurement element (10) is in the reference measurement position.

10. An opto-mechanical analysis device (1) according to any one of the preceding claims, wherein an optical damping element (12), in particular in the form of a neutral density glass, is provided and is configured to damp the first light before entering the optical reference measurement element (10) in its reference measurement position, wherein the optical damping element (12) is attached directly to the optical reference measurement element (10) or in a filter wheel (13) outside the measurement chamber (7), which filter wheel (13) is irradiated by the first light.

11. An opto-mechanical analysis device (1) according to any one of the preceding claims, wherein the optical reference measurement element (10) is formed from a glass-ceramic medium or comprises a glass-ceramic medium and is in particular Zerodur^{®}.

12. An opto-mechanical analysis device (1) according to any one of the preceding claims, wherein the first light has a beam diameter of at least 2 mm before it enters the optical reference measurement element (10) in its reference measurement position.

13. An opto-mechanical analysis device (1) according to any one of the preceding claims, wherein the control device (9) is configured to control the displacement unit (11) such that the latter pivots the optical reference measurement element (10) at regular intervals from the parking position into the reference measurement position, wherein the control device (9) is further configured to compare reference measurement data which were generated at different points in time with one another and, in the event that a deviation is above a threshold value, to output a signal locally and/or to a higher-ranking control apparatus.

## Revendications

1. Appareil d'analyse opto-mécanique (1) pour déterminer des particules fines (2) dans un gaz de mesure (3), l'appareil d'analyse opto-mécanique (1) comprenant au moins une première source de lumière (4a), une chambre de mesure (7) ayant une entrée (7a) et une sortie (7b), une pluralité de détecteurs (8), un dispositif de commande (9), un élément de mesure optique de référence (10) et une unité de déplacement (11),
dans lequel
le gaz de mesure (3) chargé en particules fines (2) peut être amené dans la chambre de mesure (7) par l'entrée (7a) et être évacué par la sortie (7b), ladite au moins une première source de lumière (4a) étant conçue pour générer une première lumière ayant une première longueur d'onde, la première lumière pouvant être injectée dans la chambre de mesure (7),
la pluralité de détecteurs (8) sont disposés à distance les uns des autres sur la chambre de mesure (7) et sont conçus pour recevoir la lumière diffusée qui se produit lorsque la première lumière frappe les particules fines (2), et pour générer des données de mesure de la lumière diffusée et pour les transmettre au dispositif de commande (9), l'unité de déplacement (11) étant conçue pour déplacer l'élément de mesure optique de référence (10) d'une position de stationnement vers une position de mesure de référence, la première lumière pouvant être injectée dans l'élément de mesure optique de référence (10) dans la position de mesure de référence, et
la pluralité de détecteurs (8) sont conçus pour recevoir la lumière diffuse provenant de l'élément de mesure optique de référence (10) dans la position de mesure de référence et pour générer des données de mesure de référence et les transmettre au dispositif de commande (9),
l'appareil d'analyse opto-mécanique (1) comprend un couvercle de fermeture (22) disposé sur l'élément de mesure optique de référence (10), le couvercle de fermeture (22) fermant l'entrée (7a) de la chambre de mesure (7) dans la position de mesure de référence,
**caractérisé en ce que** l'unité de déplacement (11) comporte un mécanisme à articulations multiples et déplace l'élément de mesure de référence (10) sur une trajectoire courbe.

2. Appareil d'analyse opto-mécanique (1) selon la revendication 1, dans lequel l'unité de déplacement (11) est conçue pour faire tourner l'élément de mesure optique de référence (10) d'un angle compris entre 60° et 120°, et en particulier de 90°, entre la position de stationnement et la position de mesure de référence.

3. Appareil d'analyse opto-mécanique (1) selon l'une des revendications précédentes,
dans lequel l'unité de déplacement (11) comprend un mécanisme à articulations multiples (19) présentant au moins une liaison à articulations multiples (27a, 27b), un moteur d'entraînement (20) et un arbre d'entraînement (21), le moteur d'entraînement (20) étant couplé en rotation à l'arbre d'entraînement (21),
une première extrémité de ladite au moins une liaison à articulations multiples (27a, 27b) est couplée en rotation à l'arbre d'entraînement (21), et l'élément de mesure optique de référence (20) est disposé à une deuxième extrémité de ladite au moins une liaison à articulations multiples (27a, 27b), un mouvement de rotation du moteur d'entraînement (20) dans une première direction entraîne le déploiement de ladite au moins une liaison à articulations multiples (27a, 27b) et le déplacement de l'élément de mesure optique de référence (10) de la position de stationnement vers la position de mesure de référence, et un mouvement de rotation du moteur d'entraînement (20) dans une deuxième direction opposée à la première direction entraîne la rétraction de ladite au moins une liaison à articulations multiples (27a, 27b) et le déplacement de l'élément de mesure optique de référence (10) de la position de mesure de référence vers la position de stationnement.

4. Appareil d'analyse opto-mécanique (1) selon la revendication 3, dans lequel l'unité de déplacement (11) comprend une unité de détection (29), en particulier sous la forme d'une barrière lumineuse, qui est conçue pour détecter si l'élément de mesure optique de référence (10) a atteint la position de stationnement ou la position de mesure de référence, l'unité de détection (29) étant conçue pour émettre un signal de détection correspondant au dispositif de commande (9) et/ou au moteur d'entraînement (20) afin d'arrêter le moteur d'entraînement (20).

5. Appareil d'analyse opto-mécanique (1) selon la revendication 3 ou 4, dans lequel un engrenage autobloquant (29), en particulier sous la forme d'un engrenage à vis sans fin, est disposé entre le moteur d'entraînement (20) et l'arbre d'entraînement (21), moyennant quoi l'élément de mesure optique de référence (10) reste dans sa position de stationnement ou dans sa position de mesure de référence lorsque le moteur d'entraînement (20) est désactivé.

6. Appareil d'analyse opto-mécanique (1) selon l'une des revendications précédentes,
dans lequel l'appareil d'analyse opto-mécanique (1) comprend un boîtier de protection (24) qui délimite un espace de réception (25), l'élément de mesure optique de référence (10) étant disposé dans l'espace de réception (25) pendant la position de stationnement.

7. Appareil d'analyse opto-mécanique (1) selon la revendication 4,
dans lequel est prévue une unité de surpression conçue pour générer une pression d'air, élevée par rapport à la pression ambiante, dans l'espace de réception (25) du boîtier de protection (24).

8. Appareil d'analyse opto-mécanique (1) selon l'une des revendications 6 ou 7,
dans lequel le boîtier de protection (24) présente une ouverture à travers laquelle l'élément de mesure optique de référence (10) peut être déployé de l'espace de réception (25) et être rétracté dans l'espace de réception (25), le couvercle de fermeture (22) fermant l'ouverture du boîtier de protection (24) dans la position de stationnement.

9. Appareil d'analyse opto-mécanique (1) selon l'une des revendications précédentes,
dans lequel est prévue une unité de nettoyage conçue pour insuffler de l'air de rinçage, en particulier filtré, dans la chambre de mesure (7) lorsque l'élément de mesure optique de référence (10) se trouve dans la position de mesure de référence.

10. Appareil d'analyse opto-mécanique (1) selon l'une des revendications précédentes,
dans lequel est prévu un élément d'atténuation optique (12), en particulier sous la forme d'un verre à densité neutre, conçu pour atténuer la première lumière avant qu'elle n'entre dans l'élément de mesure optique de référence (10) dans sa position de mesure de référence, l'élément d'atténuation optique (12) étant monté directement sur l'élément de mesure optique de référence (10) ou dans une roue à filtres (13) à l'extérieur de la chambre de mesure (7), qui est traversée par la première lumière.

11. Appareil d'analyse opto-mécanique (1) selon l'une des revendications précédentes,
dans lequel l'élément de mesure optique de référence (10) est formé d'un milieu vitrocéramique ou comprend un milieu vitrocéramique, et est en particulier du Zerodur ^{®}.

12. Appareil d'analyse opto-mécanique (1) selon l'une des revendications précédentes,
dans lequel la première lumière présente un diamètre de faisceau d'au moins 2 mm avant d'entrer dans l'élément de mesure optique de référence (10) dans sa position de mesure de référence.

13. Appareil d'analyse opto-mécanique (1) selon l'une des revendications précédentes,
dans lequel le dispositif de commande (9) est conçu pour commander l'unité de déplacement (11) de telle sorte que celle-ci fasse pivoter l'élément de mesure optique de référence (10) à intervalles réguliers de la position de stationnement vers la position de mesure de référence, le dispositif de commande (9) étant en outre conçu pour comparer entre elles des données de mesure de référence générées à différents moments et, dans le cas où un écart est supérieur à une valeur seuil, pour émettre un signal localement et/ou vers un dispositif de commande supérieur.
